# EUROPÄISCHE PATENTSCHRIFT

(11) **EP 0 390 099 B1**
(45) Veröffentlichungstag und Bekanntmachung des Hinweises auf die Patenterteilung: **26.06.1996**
(21) Anmeldenummer: 90105881.8
(22) Anmeldetag: 28.03.1990
(51) Int. Cl.: C07D 501/46, A61K 31/545

(54) **Polare Cephalosporinderivate und Verfahren zu ihrer Herstellung**
Polar cephalosporin derivatives and process for their preparation
Céphalosporines polaires et leur procédé de préparation

(30) Priorität: 31.03.1989 DE 3910421
(43) Veröffentlichungstag der Anmeldung: 03.10.1990
(73) Patentinhaber: HOECHST AKTIENGESELLSCHAFT, D-65926 Frankfurt am Main (DE)
(72) Erfinder: Kirrstetter, Reiner, Dr., D-6233 Kelkheim/Taunus (DE); Dürckheimer, Walter, Dr., D-6234 Hattersheim am Main (DE); Lattrell, Rudolf, Dr., D-6240 Königstein/Taunus (DE); Seibert, Gerhard, Prof.-Dr., D-6100 Darmstadt (DE)

(56) Entgegenhaltungen:
- EP-A- 0 111 934
- EP-A- 0 111 935
- GB-A- 2 105 334
- GB-A- 2 105 335
- US-A- 4 394 503

## Beschreibung

Die Erfindung betrifft neue polare Cephalosporinderivate, die in 3-Stellung des Cephemrings durch einen Alkylthio-Chinolinummethyl- oder Alkylthio-Isochinoliniummethylrest substituiert sind und Verfahren zu ihrer Herstellung.

Cephalosporinderivate, die antibakterielle Wirkungen haben und die in 3-Stellung des Cephemrings durch einen Chinolinummethyl- oder Isochinolinummethylrest substituiert sind, werden in den Patentdokumenten GB 2105334, GB 2105335, EP 111934 und EP 111935 beschrieben. In GB 2105334 und GB 2105335 ist der Chinoliniummethyl- oder Isochinoliniummethylrest durch einen Amino-, Alkylamino-, Di(alkyl)amino-, Hydroxyl-, Halo-, Cyano-, Trifluoromethyl-, Sulfo-, Aminosulfonyl-, Carboxy-, Alkoxycarbonyl-, Carbamoyl-, Thiocarbamoyl-, Hydroxy- substituiert Alkyl-, Formyl- oder Alkanoylrest substituiert. In EP 111934 und EP 111935 ist der Chinolinummethyl- oder Isochinoliniummethylrest durch einen Alkyl-, Alkoxy-, Halogen-, Trifluoromethyl- oder Hydroxyrest substituiert.

US-A-4 394 503 beschreibt Cephalosporinderivate gegen bakterielle Infektionen die in 3-Stellung des Cephemrings durch einen Pyridiniummethylrest substituiert sind, wobei der Pyridiniummethylrest durch einen Alkylthiorest substituiert ist.

Die erfindungsgemäßen Verbindungen zeigen sehr gute antimikrobielle Wirkung gegen gram-positive und gram-negative Bakterien und sind deshalb als Arzneimittel zur Behandlung von mikrobiellen Infektionen geeignet.

Gegenstand der Erfindung sind Cephemderivate der allgemeinen Formel I 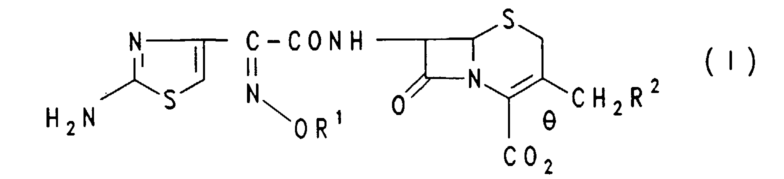 und deren physiologisch verträgliche Säureadditionssalze, worin bedeuten
- R¹: 1. Wasserstoff, 2. C₁-C₆-Alkyl, wobei der Alkylrest ein- oder mehrfach substituiert sein kann durch Halogen, C₁-C₆-Alkylthio, C₁-C₆-Alkyloxy, Aryl oder Heteroaryl, 3. C₂-C₆-Alkenyl, das ein- oder mehrfach substituiert sein kann durch Halogen, 4. C₂-C₆-Alkinyl, 5. C₃-C₇-Cycloalkyl, 6. C₃-C₇-Cycloalkyl-C₁-C₆-alkyl, 7. C₄-C₇-Cycloalkenyl, oder 8. die Gruppe 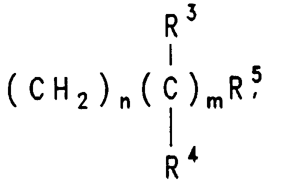 worin m und n jeweils 0 oder 1, R³ und R⁴ gleich oder verschieden sein können und Wasserstoff, Aryl, eine C₁-C₄-Alkylgruppe, oder zusammen mit dem Kohlenstoff, an das sie gebunden sind, eine Methylen- oder eine C₃-C₇-Cycloalkylidengruppe bilden; R⁵ eine Gruppe -CO₂R⁶, in der R⁶ Wasserstoff, C₁-C₄-Alkyl, -CH₂OC₁-C₄-Alkyl, -CH₂OOC-C₁-C₄-Alkyl, oder ein Equivalent eines Alkalimetalls, Erdalkalimetalls, Ammonium oder einer organischen Aminbase bedeutet;
- R²: einen Alkylthio-Chinolinium oder Alkylthio-Isochinoliniumrest, der zusätzlich noch durch C₁-C₆-Alkyl, Hydroxy-C₁-C₄-alkyl, C₁-C₄-Alkyloxy-C₁-C₄-alkyl, C₁-C₆-Alkoxy, Halogen, Trifluormethyl oder Hydroxy substituiert sein kann, und in der die R¹O-Gruppe in syn-Position steht.

Die vorliegende Erfindung ist insbesondere auf Verbindungen gerichtet, in denen die Substituenten die folgende Bedeutung besitzen:
- R¹: Wasserstoff, C₁-C₆-Alkyl, vorzugsweise C₁-C₄-Alkyl wie z.B. Methyl, Ethyl, Propyl, Isopropyl, Butyl, vorzugsweise Methyl und Ethyl, insbesondere Methyl, wobei der Alkylrest ein- oder mehrfach substituiert sein kann durch Halogen, C₁-C₆-Alkylthio, C₁-C₆-Alkyloxy, Aryl oder Heteroaryl, C₂-C₆-Alkenyl, das ein- oder mehrfach substituiert sein kann durch Halogen, C₂-C₃-Alkinyl, C₃-C₇-Cycloalkyl, C₃-C₇-Cycloalkyl-C₁-C₆-alkyl, C₄-C₇-Cycloalkenyl, und in der Gruppe 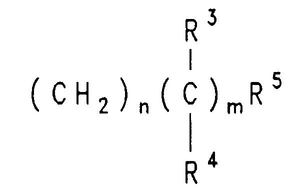 die vorstehende Bedeutung besitzt, wobei auch bei diesen bevorzugten Verbindungen die R¹O-Gruppe in syn Position steht.
- R²: einen Alkylthio-Chinoliniumrest 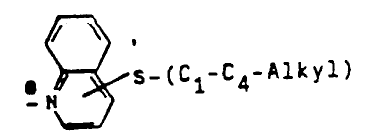 der zusätzlich noch durch andere Gruppen, vorzugsweise 1- bis 3-fach, insbesondere 1- bis 2-fach, gleich oder verschieden substituiert sein kann, durch C₁-C₄-Alkyl, wie insbesondere Methyl, Ethyl, Propyl, Isopropyl, Butyl, Hydroxy-C₁-C₄-alkyl, insbesondere Hydroxymethyl, C₁-C₄-Alkyloxy-C₁-C₄-alkyl, insbesondere Methoxymethyl; durch C₁-C₆-Alkoxy, beispielsweise Ethoxy, Methoxy; durch Halogen, wie z.B. Fluor, Chlor, Brom, Iod; durch Trifluormethyl oder Hydroxy;
oder einen Alkylthio-Isochinoliniumrest 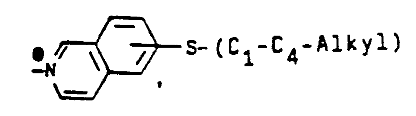 der wie vorstehend der Chinoliniumrest noch zusätzlich substituiert sein kann.

Unter Alkylthio ist also insbesondere ein C₁-C₄-Alkylthio zu verstehen, wie z.B. Methyl-, Ethyl-, Propyl-, Isopropyl-, n-Butyl- oder sec-Butyl-thio, vorzugsweise Methyl- und Ethyl-, insbesondere Methylthio. Bevorzugte Beispiele für R² sind beispielsweise die folgenden:
2-, 3-, 4-, 5-, 6-, 7- oder 8-Methylthiochinolinium;
2-, 3-, 4-, 5-, 6-, 7- oder 8-Ethylthiochinolinium;
4-Methylthio-7-trifluormethylchinolinium; 1-, 3-, 4-, 5-, 6-, 7- oder 8-Methylthioisochinolinium und 1-, 3-, 4-, 5-, 6-, 7- oder 8-Ethylthioisochinolinium.

Erfindungsgemäß besonders bevorzugte Verbindungen sind demnach solche, in denen R¹ für Methyl oder Ethyl und R² für Methyl- oder Ethylthiochinolinium und Methyl- oder Ethylthioisochinolinium steht, wobei die Methylthioreste noch bevorzugt sind.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man eine Verbindung der allgemeinen Formel II 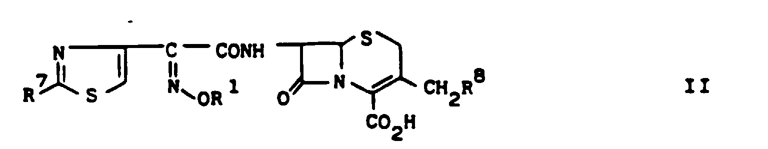 oder deren Salze oder ein reaktionsfähiges Derivat der Verbindung II, worin R¹ die obengenannte Bedeutung hat und R⁷ eine Amino- oder eine geschützte Aminogruppe bedeutet und R⁸ eine durch Chinoline oder Isochinoline, die den Resten R² der Formel I entsprechen, austauschbare Gruppe bedeutet, mit einem solchen Chinolin oder Isochinolin umsetzt und
a) eine gegebenenfalls vorhandene Schutzgruppe abspaltet und
b) falls erforderlich, das erhaltene Produkt in ein physiologisch verträgliches Säureadditionssalz überführt.

Soll die Herstellung der Verbindungen der allgemeinen Formel I durch nucleophilen Austausch von R⁸ in den Verbindungen der allgemeinen Formel II durch dem Rest R² entsprechende Chinoline oder Isochinoline erfolgen, so kommen als Reste R⁸ insbesondere in Betracht Acyloxyreste von niederen aliphatischen Carbonsäuren, vorzugsweise mit 1 - 4 C-Atomen, wie z.B. Acetoxy oder Propionyloxy, insbesondere Acetoxy, die gegebenenfalls substituiert sein können, wie z.B. Chloracetoxy oder Acetylacetoxy. Erfindungsgemäß werden bei der nucleophilen Austauschreaktion bevorzugt Ausgangsverbindungen der allgemeinen Formel II, in denen R⁸ für Acetoxy steht, eingesetzt.

Liegt die Gruppe R⁷ als geschützte Aminofunktion vor, so eignen sich als Aminoschutzgruppen z.B. gegebenenfalls substituiertes Alkyl, wie beispielsweise tert.-Butyl, tert.-Amyl, Benzyl, p-Methoxybenzyl, Trityl, Benzhydryl, vorzugsweise Trityl; Trialkylsilyl, wie beispielsweise Trimethylsilyl; gegebenenfalls substituiertes aliphatisches Acyl, wie z.B. Formyl, Chloracetyl, Bromacetyl, Trichloracetyl und Trifluoracetyl, vorzugsweise Formyl; oder gegebenenfalls substituiertes Alkoxycarbonyl wie beispielsweise Trichlorethoxycarbonyl, Benzyloxycarbonyl oder tert.-Butyloxycarbonyl, vorzugsweise tert.-Butyloxycarbonyl und Benzyloxycarbonyl; oder Dimethylaminomethylen.

Die Schutzgruppe kann nach der Austauschreaktion in an sich bekannter Weise abgespalten werden, z.B. die Tritylgruppe mittels einer Carbonsäure, wie z.B. Essigsäure, Trifluoressigsäure, Ameisensäure oder die Benzyloxycarbonylgruppe hydrogenolytisch.

Die Reaktionsprodukte der Formel I können aus der Reaktionsmischung in üblicher Weise, z.B. durch Gefriertrocknen der Wasserphase, Chromatographie oder auch durch Ausfällen als schwerlösliches Salz, beispielsweise als Hydroiodid- oder Hydrothiocyanatsalz, isoliert werden.

Die nucleophile Austauschreaktion an Verbindungen der allgemeinen Formel II erfolgt so, daß die Reaktion in organischem Lösungsmittel vorgenommen wird, wobei das Ausgangsmaterial II zunächst mit einem Silylierungsmittel und Trimethyliodsilan behandelt wird und nachfolgend mit einem silylierten Chinolin- bzw. Isochinolinderivat zur Reaktion gebracht wird. Geeignete Lösungsmittel sind chlorierte Kohlenwasserstoffe, wie Methylenchlorid, Chloroform, Dichlorethan, Trichlorethan, Tetrachlorkohlenstoff, oder Niederalkylnitrile, wie Acetonitril oder Propionitril oder Frigene; insbesondere sind Methylenchlorid und Chloroform hervorragende Lösungsmittel. Geeignete Silylierungsmittel sind z.B. Bistrimethylsilylacetamid, N-Methyl-N-trimethylsilyltrifluoracetamid; Bistrimethylsilyltrifluoracetamid, Trimethylchlorsilan, Hexamethyldisilazan, Bistrimethylsilylharnstoff. Die Reaktion wird bei Temperaturen zwischen -5°C und +40°C, vorzugsweise bei Raumtemperatur durchgeführt. Die Reaktionsprodukte der Formel I können nach Zugabe von Wasser als Hydroiodidsalze isoliert und durch literaturbekannte Chromatographie an Kieselgel gereinigt werden.

Als physiologisch verträgliche Säureadditionssalze der Verbindungen der allgemeinen Formel I seien beispielsweise erwähnt solche mit Chlorwasserstoffsäure, Phosphorsäure, Schwefelsäure oder organischen Säuren, wie z.B. Methansulfonsäure, p-Toluolsulfonsäure oder Maleinsäure.

Die erfindungsgemäß erhaltenen Verbindungen der allgemeinen Formel I und ihre physiologisch verträglichen Säureadditionssalze zeigen bemerkenswert hohe antibakterielle Wirksamkeit sowohl gegen grampositive als auch gramnegative bakterielle Keime. Auch gegen penicillinase- und cephalosporinase-bildende Bakterien sind die Verbindungen der Formel I unerwartet gut wirksam.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen. Die Verbindungen der allgemeinen Formel I und ihre physiologisch verträglichen Säureadditionssalze können intramuskulär oder intravenös verabfolgt werden.

Geeignete Dosen der Verbindungen der allgemeinen Formel I oder ihrer physiologisch verträglichen Säureadditionssalze liegen bei etwa 0,2 bis 10 g/Tag, vorzugsweise bei 0,5 bis 4 g/Tag, für einen Erwachsenen von etwa 60 kg Körpergewicht. Es können Einzel- oder im allgemeinen Mehrfachdosen verabreicht werden, wobei die Einzeldosis den Wirkstoff in einer Menge von etwa 100 bis 2000 mg, vorzugsweise von etwa 250 bis 1000 mg, enthalten kann.

Die folgenden Ausführungsbeispiele für erfindungsgemäß herstellbare syn-Verbindungen dienen zur weiteren Erläuterung der Erfindung, schränken sie jedoch nicht darauf ein.

### Beispiel 1

### 3- [(2-Methylthio-1-chinolinium)-methyl]-6R,7R-7- [2-(2-aminothiazol-4-yl)-2-syn-methoxyiminoacetamido]-3-cephem-4-carboxylat

Zu einer Suspension von 4,5 g (10 mmol) 6R,7R-7-[2-Aminothiazol-4-yl)-2-syn-methoxyiminoacetamido]-3-acetoxymethyl -3-cephem-4-carbonsäure in 20 ml Chloroform werden 6,3 ml N-Methyl-N-trimethylsilyltrifluoracetamid (MSTFA) zugegeben und 1 h bei Raumtemp, gerührt. Nach Zugabe von 3,8 ml Trimethyliodsilan wird weitere 15 min gerührt und dann i. V. zur Trockene eingedampft. Der Rückstand wird in 20 ml Acetonitril aufgenommen, mit 0,8 ml Tetrahydrofuran versetzt und zu einer Mischung aus 2,16 g 2-Methylthiochinolin und 4,2 ml MSTFA in 10 ml Acetonitril gegeben und danach 2,5 h bei Raumtemperatur gerührt. Durch Zusatz von 1,2 ml Wasser wird das gewünschte Produkt als Hydroiodid ausgefällt (4.6 g), das dann zur weiteren Reinigung an Kieselgel mit Aceton/Wasser (3:1) chromatographiert wird. Nach dem Einengen und Gefriertrocknen erhält man 250 mg der Titelverbindung.

¹H-NMR (CF₃CO₂D): δ = 2.80(3H, s, SCH₃), 3.40 und 3.67(2H, AB, J=18Hz, SCH₂), 4.26(3H, s, OCH₃), 5.40(1H, d, J=5Hz, 6-H), 6.10(1H, d, J=5Hz, 7-H), 6.14 und 6.23(2H, AB, J=16Hz, 3-CH₂), 7.41(1H, s, Thiazol-H), 7.74-8.35(4H, m, Chinolin-H), 9.21 und 9.62(2H, AB, J=8Hz, Chinolin-H).

### Bildung eines physiologisch verträglichen Säureadditionssalzes (Sulfat des Beispiels 1)

Zu einer Mischung von 6 ml Wasser, 6 ml Toluol und 2 g Ionenaustauscher LA 2 (wasserunlösliches sekundäres Amin) werden 1,8 g des voran beschriebenen Hydroiodids eingetragen und 30 min gerührt, wobei das Produkt in Lösung geht. Nach Absaugen über eine Seitz-Filterschicht werden die Phasen getrennt. Die wäßrige Phase wird mit 6 ml Toluol gewaschen und mit 100 mg Aktivkohle für 15 min gerührt.

Nach Absaugen über einen Klärschichtfilter wird mit 20 %iger H₂SO₄ auf pH 1.3 gestellt, und durch Zugabe von 14 ml Ethanol wird das Sulfat-Addukt von Beispiel 1 bei 0-5°C ausgefällt. Nach Abfiltration und Trocknung erhält man 200 mg der Titelverbindung als Sulfat-Addukt.

Die in den Beispielen 2-8 beschriebenen Verbindungen werden unter Verwendung der jeweiligen literaturbekannten Chinolin- oder Isochinolin-Derivate in analoger Weise hergestellt.

### Beispiel 2

### 3- [(3-Methylthio-1-chinolinium)-methyl] -6R,7R-7- [2-(2-aminothiazol-4-yl)-2-syn-methoxyiminoacetamido] -3-cephem-4-carboxylat

¹H-NMR (CF₃CO₂D) : δ = 2.80(3H, s, SCH₃), 3.41 und 3.68 (2H, AB, J=18Hz, SCH₂), 4.25(3H, s, OCH₃), 5.43(1H, d, J=5Hz, 6-H), 6.12(1H, d, J=5Hz, 7-H), 6.14 und 6.43 (2H, AB, J=16Hz, 3-CH₂), 7.44(1H, s, Thiazol-H), 8.0-8.45(4H, m, Chinolin-H), 8.82(1H, br s, Chinolin-H), 9.13(1H, br s, Chinolin-H).

### Beispiel 3

### 3- [(4-Methylthio-1-chinolinium)-methyl] -6R,7R-7- [2-(2-aminothiazol-4-yl)-2-syn-methoxyiminoacetamido] -3-cephem-4-carboxylat

¹H-NMR (CF₃CO₂D): δ = 2.83(3H, s, SCH₃), 3.36 und 3.54(2H, AB, J=18Hz, SCH₂), 4.20(3H, s, OCH₃), 5.36(1H, d, J=5Hz, 6-H), 5.94(1H, d, J=5Hz, 7-H), 6.04 und 6.15 (2H, AB, J= 16Hz, 3-CH₂), 7.37(1H, s, Thiazol-H), 7.55-8.90(6H, m, Chinolin-H).

### Beispiel 4

### 3- [(5-Methylthio-1-chinolinium)-methyl] -6R,7R-7- [2-(2-aminothiazol-4-yl)-2-syn-methoxyiminoacetamido] -3-cephem-4-carboxylat

¹H-NMR (CF₃CO₂D): δ = 2.80(3H, s, SCH₃), 3.43 und 3.72(2H, AB, J=18Hz, SCH₂), 4.26(3H, s, OCH₃), 5.44(1H. d, J=5Hz, 6-H), 6.12(1H, d, J=5Hz, 7-H), 6.06 und 6.39(2H, AB, J=18Hz, 3-CH₂), 7.42(1H, s, Thiazol-H), 7.80-8.25(4H, m, Chinolin-H), 9.23(1H, d, J=6Hz, Chinolin-H), 9.60(1H, d, J=9Hz, Chinolin-H).

### Beispiel 5

### 3- [(6-Methylthio-1-chinolinium)-methyl]-6R,7R-7-[2-(2-aminothiazol-4-yl)-2-syn-methoxyiminoacetamido] -3-cephem-4-carboxylat

¹H-NMR (CF₃CO₂D) : δ = 2.69(3H, s, SCH₃), 3.48 und 3.67(2H, AB, J=18Hz, SCH₂), 4.22(3H, s, OCH₃), 5.40(1H, d, J=5Hz, 6-H), 6.07(1H, d, J=5Hz, 7-H), 5.90 und 6.38(2H, AB, J=16Hz, 3-CH₂), 7.38(1H, s, Thiazol-H), 7.80-8.40(4H, m, Chinolin-H), 8.80-9.15 (2H, m, Chinolin-H).

### Beispiel 6

### 3-[(4-Methylthio-7-trifluormethyl-1-chinolinium)-methyl]-6R,7R-7-[2-(2-aminothiazol-4-yl)-2-syn-methoxyiminoacetamido]-3-cephem-4-carboxylat

¹H-NMR (CF₃CO₂D): δ = 2.93(3H, s, SCH₃), 3.45 und 3.73 (2H, AB, J=18Hz, SCH₂), 4.23(3H, s, OCH₃), 5.40(1H, d, J=5Hz, 6-H), 6.07(1H, d, J=5Hz, 7-H), 5.92 und 6.28(2H, AB, J=16Hz, 3-CH₂), 7.38(1H, s, Thiazol-H), 7.60-9.00(5H, m Chinolin-H).

### Beispiel 7

### 3- [(5-Methylthio-2-isochinolinium)-methyl]-6R,7R-7-[2-(2-aminothiazol-4-yl)-2-syn-methoxyiminoacetamido] -3-cephem-4-carboxylat

¹H-NMR (CF₃CO₂D): δ =2.76(3H, s, SCH₃), 3.51 und 3.91(2H, AB, J=18Hz, SCH₂), 4.22(3H, s, OCH₃), 5.43(1H, d, J=5Hz, 6-H), 6.15(1H, d, J=5Hz, 7-H), 5.56 und 6.38 (2H, AB, J=15Hz, 3-CH₂), 7.43(1H, s, Thiazol-H), 8.0-8.25(3H, m, Isochinolin-H), 8.72 und 8.80(2H, AB, J=7Hz, Isochinolin-H), 9.77(1H, s, Isochinolin-H).

### Beispiel 8

### 3- [(5-Ethylthio-2-isochinolinium)methyl] -6R,7R-7- [2-(2-aminothiazol-4-yl)-2-syn-methoxyiminoacetamido] -3-cephem-4-carboxylat

¹H-NMR (CF₃CO₂D): δ = 1.44(3H, t, J=7Hz, CH₃), 3.19(2H, q, J=7Hz, S-CH₂-CH₃), 3.43 und 3.90(2H, AB, J=18Hz, SCH₂), 4.35(3H, s, OCH₃), 5.35(1H, d, J=5Hz, 6-H), 5.96 (1H, d, J=5Hz, 7-H), 5.50 und 6.27(2H, AB, J=15Hz, 3-CH₂), 7.38(1H, s, Thiazol-H), 7.90-8.45(3H, m, Isochinolin-H), 8.62 und 8.88(2H, AB, J=7Hz, Isochinolin-H), 9.70(1H, br s, Isochinolin-H).

## Patentansprüche (Patentansprüche für folgende(n) Vertragsstaat(en): AT, BE, CH, DE, DK, FR, GB, IT, LI, LU, NL, SE)

1. Polare Cephalosporinderivate der allgemeinen Formel I 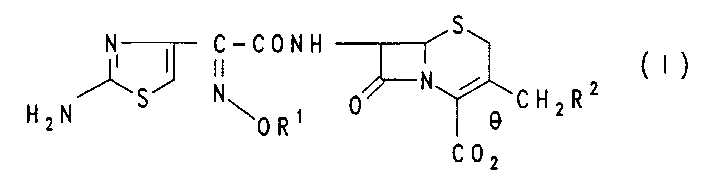 und deren physiologisch verträgliche Säureadditionssalze, worin bedeuten
R¹ 1. Wasserstoff, 2. C₁-C₆-Alkyl, wobei der Alkylrest ein- oder mehrfach substituiert sein kann durch Halogen, C₁-C₆-Alkylthio, C₁-C₆-Alkyloxy, Aryl oder Heteroaryl, 3. C₂-C₆-Alkenyl, das ein- oder mehrfach substituiert sein kann durch Halogen, 4. C₂-C₆-Alkinyl, 5. C₃-C₇-Cycloalkyl, 6. C₃-C₇-Cycloalkyl-C₁-C₆-alkyl, 7. C₄-C₇-Cycloalkenyl, oder 8. die Gruppe 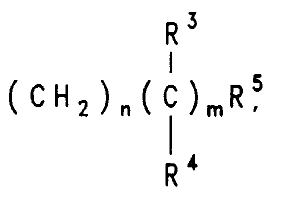 worin m und n jeweils 0 oder 1, R³ und R⁴ gleich oder verschieden sein können und Wasserstoff, Aryl, eine C₁-C₄-Alkylgruppe, oder zusammen mit dem Kohlenstoff, an das sie gebunden sind, eine Methylen- oder eine C₃-C₇-Cycloalkylidengruppe bilden; R⁵ eine Gruppe -CO₂R⁶, in der R⁶ Wasserstoff, C₁-C₄-Alkyl, -CH₂OC₁-C₄-Alkyl, -CH₂OOC-C₁-C₄-Alkyl, oder ein Equivalent eines Alkalimetalls, Erdalkalimetalls, Ammonium oder einer organischen Aminbase bedeutet;
R² einen Alkylthio-Chinolinium oder Alkylthio-lsochinoliniumrest, der zusätzlich noch durch C₁-C₆-Alkyl, Hydroxy-C₁-C₄-alkyl, C₁-C₄-Alkyloxy-C₁-C₄-alkyl, C₁-C₆-Alkoxy, Halogen, Trifluormethyl oder Hydroxy substituiert sein kann, und in der die R¹O-Gruppe in syn-Position steht.

2. Verfahren zur Herstellung von Verbindungen der Formel I und ihrer physiologisch verträglichen Säureadditionssalze, dadurch gekennzeichnet, daß man eine Verbindung der allgemeinen Formel II 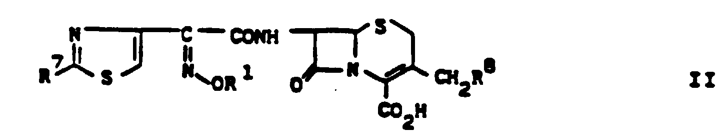 oder deren Salze oder ein reaktionsfähiges Derivat der Verbindung II, worin R¹ die obengenannte Bedeutung hat und R⁷ eine Amino- oder eine geschützte Aminogruppe bedeutet und R⁸ eine durch Chinoline oder Isochinoline, die den Resten R² der Formel I entsprechen, austauschbare Gruppe bedeutet, mit einem solchen Chinolin oder Isochinolin umsetzt und
a) eine gegebenenfalls vorhandene Schutzgruppe abspaltet und
b) falls erforderlich, das erhaltene Produkt in ein physiologisch verträgliches Säureadditionssalz überführt.

3. Gegen bakterielle Infektionen wirksame pharmazeutische Zubereitungen, gekennzeichnet durch einen Gehalt an Cephalosporinderivaten der allgemeinen Formel I oder deren physiologisch verträglichen Säureadditionssalzen.

4. Verfahren zur Herstellung von gegen bakterielle Infektionen wirksamen pharmazeutischen Zubereitungen, dadurch gekennzeichnet, daß ein Cephalosporinderivat der allgemeinen Formel I mit pharmazeutisch üblichen Trägerstoffen oder Verdünnungsmitteln in eine pharmazeutisch geeignete Verabreichungsform gebracht wird.

5. Verwendung von Cephalosporinderivaten der allgemeinen Formel I zur Herstellung von Arzneimitteln zur Bekämpfung bakterieller Infektionen.

## Patentansprüche (Patentansprüche für folgende(n) Vertragsstaat(en): ES, GR)

1. Verfahren zur Herstellung von polaren Cephalosporinderivaten der allgemeinen Formel I 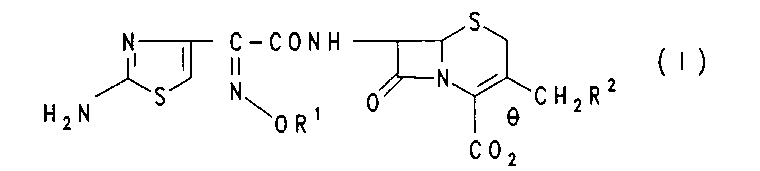 und deren physiologisch verträglichen Säureadditionssalze, worin bedeuten
R¹ 1. Wasserstoff, 2. C₁-C₆-Alkyl, wobei der Alkylrest ein- oder mehrfach substituiert sein kann durch Halogen, C₁-C₆-Alkylthio, C₁-C₆-Alkyloxy, Aryl oder Heteroaryl, 3. C₂-C₆-Alkenyl, das ein- oder mehrfach substituiert sein kann durch Halogen, 4. C₂-C₆-Alkinyl, 5. C₃-C₇-Cycloalkyl, 6. C₃-C₇-Cycloalkyl-C₁-C₆-alkyl, 7. C₄-C₇-Cycloalkenyl, oder 8. die Gruppe 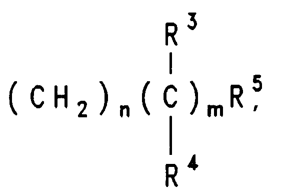 worin m und n jeweils 0 oder 1, R³ und R⁴ gleich oder verschieden sein können und Wasserstoff, Aryl, eine C₁-C₄-Alkylgruppe, oder zusammen mit dem Kohlenstoff, an das sie gebunden sind, eine Methylen- oder eine C₃-C₇-Cycloalkylidengruppe bilden; R⁵ eine Gruppe -CO₂R⁶, in der R⁶ Wasserstoff, C₁-C₄-Alkyl, -CH₂OC₁-C₄-Alkyl, -CH₂OOC-C₁-C₄-Alkyl, oder ein Equivalent eines Alkalimetalls, Erdalkalimetalls, Ammonium oder einer organischen Aminbase bedeutet;
R² einen Alkylthio-Chinolinium oder Alkylthio-lsochinoliniumrest, der zusätzlich noch durch C₁-C₆-Alkyl, Hydroxy-C₁-C₄-alkyl, C₁-C₄-Alkyloxy-C₁-C₄-alkyl, C₁-C₆-Alkoxy, Halogen, Trifluormethyl oder Hydroxy substituiert sein kann, und in der die R¹O-Gruppe in syn-Position steht,
dadurch gekennzeichnet, daß man eine Verbindung der allgemeinen Formel II 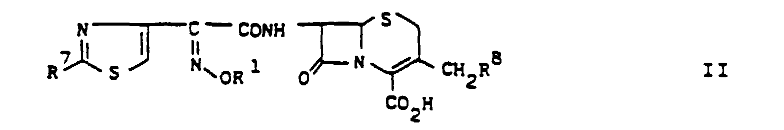 oder deren Salze oder ein reaktionsfähiges Derivat der Verbindung II, worin R¹ die obengenannte Bedeutung hat und R⁷ eine Amino- oder eine geschützte Aminogruppe bedeutet und R⁸ eine durch Chinoline oder Isochinoline, die den Resten R² der Formel I entsprechen, austauschbare Gruppe bedeutet, mit einem solchen Chinolin oder Isochinolin umsetzt und
a) eine gegebenenfalls vorhandene Schutzgruppe abspaltet und
b) falls erforderlich, das erhaltene Produkt in ein physiologisch verträgliches Säureadditionssalz überführt.

2. Verwendung von Cephalosporinderivaten der allgemeinen Formel I zur Herstellung von Arzneimitteln zur Bekämpfung bakterieller Infektionen.

## Claims (Claims for the following Contracting State(s): AT, BE, CH, DE, DK, FR, GB, IT, LI, LU, NL, SE)

1. A polar cephalosporin derivative of the formula I 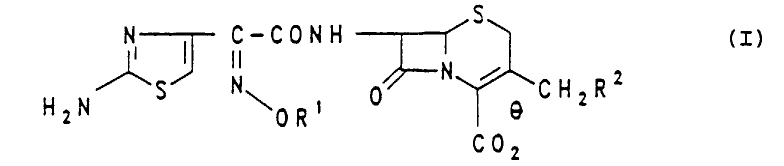 and the physiologically tolerated acid addition salts thereof, in which
R¹ is 1. hydrogen, 2. C₁-C₆-alkyl, where the alkyl radical can be mono- or polysubstituted by halogen, C₁-C₆-alkylthio, C₁-C₆-alkyloxy, aryl or heteroaryl, 3. C₂-C₆-alkenyl which can be mono- or polysubstituted by halogen, 4. C₂-C₆-alkynyl, 5. C₃-C₇-cycloalkyl, 6. C₃-C₇-cycloalkyl-C₁-C₆-alkyl, 7. C₄-C₇-cycloalkenyl, or 8. the group 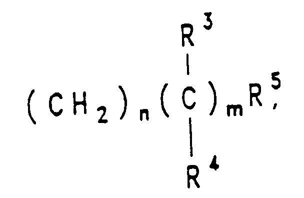 in which m and n can each be 0 or 1, R³ and R⁴ can be identical or different and are hydrogen, aryl, a C₁-C₄-alkyl group, or form, together with the carbon to which they are bonded, a methylene or a C₃-C₇-cycloalkylidene group; R⁵ is a group -CO₂R⁶, in which R⁶ is hydrogen, C₁-C₄-alkyl, -CH₂OC₁-C₄-alkyl, -CH₂OOC-C₁-C₄-alkyl, or an equivalent of an alkali metal, alkaline earth metal, ammonium or of an organic amine base;
R² is an alkylthioquinolinium or alkylthioisoquinolinium radical which can additionally be substituted by C₁-C₆-alkyl, hydroxy-C₁-C₄-alkyl, C₁-C₄-alkyloxy-C₁-c₄-alkyl, C₁-C₆-alkoxy, halogen, trifluoromethyl or hydroxyl, and in which the R¹O group is in the syn position.

2. A process for the preparation of a compound of the formula I and the physiologically tolerated acid addition salts thereof, which comprises reacting a compound of the formula II 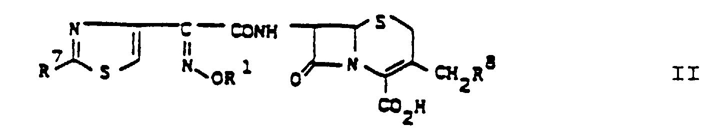 or salts thereof or a reactive derivative of the compound II, in which R¹ has the abovementioned meaning and R⁷ is an amino or a protected amino group and R⁸ is a group replaceable by quinolines or isoquinolines which correspond to the radicals R² in the formula I, with a quinoline or isoquinoline of this type, and
a) eliminating a protective group which is present where appropriate, and
b) where necessary, converting the resulting product into a physiologically tolerated acid addition salt.

3. A pharmaceutical composition effective against bacterial infections, which contain cephalosporin derivatives of the formula I or the physiologically tolerated acid addition salts thereof.

4. A process for the preparation of pharmaceutical compositions effective against bacterial infections, which comprises converting a cephalosporin derivative of the formula I with pharmaceutically customary excipients or diluents into a pharmaceutically suitable administration form.

5. The use of cephalosporin derivatives of the formula I for the preparation of pharmaceuticals for controlling bacterial infections.

## Claims (Claims for the following Contracting State(s): ES, GR)

1. A process for the preparation of a polar cephalosporin derivative of the formula I 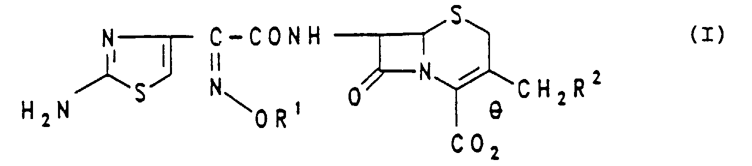 and the physiologically tolerated acid addition salts thereof, in which
R¹ is 1. hydrogen, 2. C₁-C₆-alkyl, where the alkyl radical can be mono- or polysubstituted by halogen, C₁-C₆-alkylthio, C₁-C₆-alkyloxy, aryl or heteroaryl, 3. C₂-C₆-alkenyl which can be mono- or polysubstituted by halogen, 4. C₂-C₆-alkynyl, 5. C₃-C₇-cycloalkyl, 6. C₃-C₇-cycloalkyl-C₁-C₆-alkyl, 7. C₄-C₇-cycloalkenyl, or 8. the group 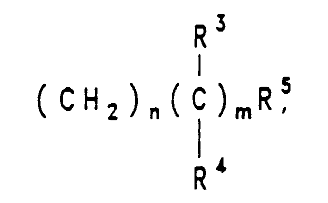 in which m and n can each be 0 or 1, R³ and R⁴ can be identical or different and are hydrogen, aryl, a C₁-C₄-alkyl group, or form, together with the carbon to which they are bonded, a methylene or a C₃-C₇-cycloalkylidene group; R⁵ is a group -CO₂R⁶, in which R⁶ is hydrogen, C₁-C₄-alkyl, -CH₂OC₁-C₄-alkyl, -CH₂OOC-C₁-C₄-alkyl, or an equivalent of an alkali metal, alkaline earth metal, ammonium or of an organic amine base;
R² is an alkylthioquinolinium or alkylthioisoquinolinium radical which can additionally be substituted by C₁-C₆-alkyl, hydroxy-C₁-C₄-alkyl, C₁-C₄-alkyloxy-C₁-C₄-alkyl, C₁-C₆-alkoxy, halogen, trifluoromethyl or hydroxyl, and in which the R¹O group is in the syn position, which comprises reacting a compound of the formula II 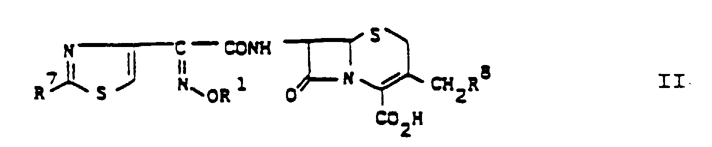
or salts thereof or a reactive derivative of the compound II, in which R¹ has the abovementioned meaning and R⁷ is an amino or a protected amino group and R⁸ is a group replaceable by quinolines or isoquinolines which correspond to the radicals R² in the formula I, with a quinoline or isoquinoline of this type, and
a) eliminating a protective group which is present where appropriate, and
b) where necessary, converting the resulting product into a physiologically tolerated acid addition salt.

2. The use of cephalosporin derivatives of the formula I for the preparation of pharmaceuticals for controlling bacterial infections.

## Revendications (Revendications pour l'(les) Etat(s) contractant(s) suivant(s): AT, BE, CH, DE, DK, FR, GB, IT, LI, LU, NL, SE)

1. Dérivés polaires de céphalosporine de formule générale I 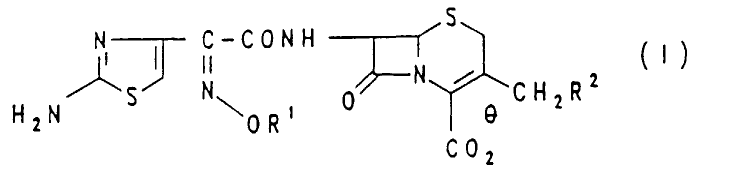 et leurs sels d'addition avec des acides physiologiquement acceptables, formule dans laquelle
R¹ représente 1. un atome d'hydrogène, 2. un radical alkyle en C₁-C₆, le radical alkyle pouvant être une ou plusieurs fois substitué par un ou des atomes d'halogène ou groupes alkyl(C₁-C₆)thio, alkyloxy en C₁-C₆, aryle ou hétéroaryle, 3. un radical alcényle en C₂-C₆ pouvant être une ou plusieurs fois substitué par un ou des atomes d'halogène, 4. un radical alcynyle en C₂-C₆, 5. un radical cycloalkyle en C₃-C₇, 6. un radical cycloalkyl(C₃-C₇)-alkyle(C₁-C₆), 7. un radical cycloalcényle en C₄-C₇, ou 8. le groupe 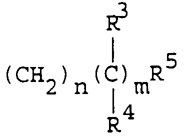 dans lequel m et n sont chacun 0 ou 1, R³ et R⁴ peuvent être identiques ou différents et représentent un atome d'hydrogène ou un groupe aryle ou alkyle en C₁-C₄, ou forment ensemble, avec l'atome de carbone auquel ils sont liés, un groupe méthylène ou cycloalkylidène en C₃-C₇; R⁵ représente un groupe -CO₂R⁶ dans lequel R⁶ représente un atome d'hydrogène ou un radical alkyle en C₁-C₄, -CH₂O-alkyle(C₁-C₄), -CH₂OOC-alkyle(C₁-C₄), ou un équivalent d'un métal alcalin, d'un métal alcalino-terreux, de l'ion ammonium ou d'une base de type amine organique;
R² représente un radical alkylthioquinolinium ou alkylthioisoquinolinium, qui peut être additionnellement substitué encore par un atome d'halogène ou par un groupe alkyle en C₁-C₆, hydroxyalkyle en C₁-C₄, alkyloxy(C₁-C₄)-alkyle-(C₁-C₄), alcoxy en C₁-C₆, trifluorométhyle ou hydroxy,
et dans laquelle le groupe R¹O est en position *syn*.

2. Procédé pour la préparation des composés de formule I et de leurs sels d'addition avec des acides physiologiquement acceptables, caractérisé en ce que l'on fait réagir un composé de formule générale II 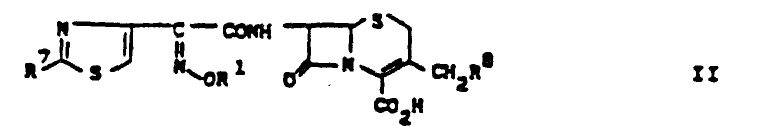 ou ses sels, ou un dérivé réactif du composé II, dans lequel R¹ a la signification donnée précédemment et R⁷ représente un groupe amino ou un groupe amino protégé, et R⁸ représente un groupe échangeable par des quinoléines ou isoquinoléines qui correspondent aux radicaux R² de la formule I, avec une telle quinoléine ou isoquinoléine, et
a) on élimine un groupe protecteur éventuellement présent et
b) si nécessaire, on convertit le produit obtenu en un sel d'addition avec un acide physiologiquement acceptable.

3. Composition pharmaceutique active contre des infections bactériennes, caractérisée par une teneur en dérivés de céphalosporine de formule générale I ou leurs sels d'addition avec des acides physiologiquement acceptables.

4. Procédé pour la préparation de compositions pharmaceutiques actives contre des infections bactériennes, caractérisé en ce que l'on met sous une forme d'administration pharmaceutiquement appropriée un dérivé de céphalosporine de formule générale I, avec des véhicules ou diluants pharmaceutiquement usuels.

5. Utilisation des dérivés de céphalosporine de formule générale I pour la fabrication de médicaments destinés à combattre des infections bactériennes.

## Revendications (Revendications pour l'(les) Etat(s) contractant(s) suivant(s): ES, GR)

1. Procédé pour la préparation de dérivés polaires de céphalosporine de formule générale I 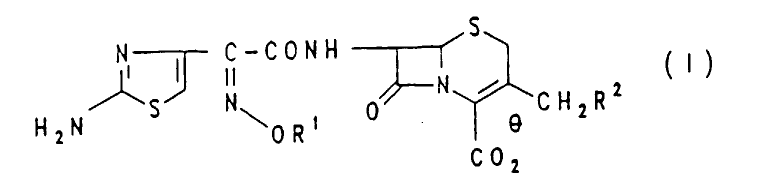 et de leurs sels d'addition avec des acides physiologiquement acceptables, formule dans laquelle
R¹ représente 1. un atome d'hydrogène, 2. un radical alkyle en C₁-C₆, le radical alkyle pouvant être une ou plusieurs fois substitué par un ou des atomes d'halogène ou groupes alkyl(C₁-C₆)thio, alkyloxy en C₁-C₆, aryle ou hétéroaryle, 3. un radical alcényle en C₂-C₆ pouvant être une ou plusieurs fois substitué par un ou des atomes d'halogène, 4. un radical alcynyle en C₂-C₆, 5. un radical cycloalkyle en C₃-C₇, 6. un radical cycloalkyl(C₃-C₇)-alkyle(C₁-C₆), 7. un radical cycloalcényle en C₄-C₇, ou 8. le groupe 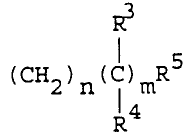 dans lequel m et n sont chacun 0 ou 1, R³ et R⁴ peuvent être identiques ou différents et représentent un atome d'hydrogène ou un groupe aryle ou alkyle en C₁-C₄, ou forment ensemble, avec l'atome de carbone auquel ils sont liés, un groupe méthylène ou cycloalkylidène en C₃-C₇; R⁵ représente un groupe -CO₂R⁶ dans lequel R⁶ représente un atome d'hydrogène ou un radical alkyle en C₁-C₄, -CH₂O-alkyle(C₁-C₄), -CH₂OOC-alkyle(C₁-C₄), ou un équivalent d'un métal alcalin, d'un métal alcalino-terreux, de l'ion ammonium ou d'une base de type amine organique;
R² représente un radical alkylthioquinolinium ou alkylthioisoquinolinium, qui peut être additionnellement substitué encore par un atome d'halogène ou par un groupe alkyle en C₁-C₆, hydroxyalkyle en C₁-C₄, alkyloxy(C₁-C₄)-alkyle-(C₁-C₄), alcoxy en C₁-C₆, trifluorométhyle ou hydroxy,
et dans laquelle le groupe R¹O est en position *syn*, caractérisé en ce que l'on fait réagir un composé de formule générale II 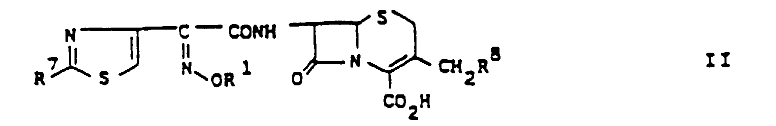 ou ses sels, ou un dérivé réactif du composé II, dans lequel R¹ a la signification donnée précédemment et R⁷ représente un groupe amino ou un groupe amino protégé, et R⁸ représente un groupe échangeable par des quinoléines ou isoquinoléines qui correspondent aux radicaux R² de la formule I, avec une telle quinoléine ou isoquinoléine, et
a) on élimine un groupe protecteur éventuellement présent et
b) si nécessaire, on convertit le produit obtenu en un sel d'addition avec un acide physiologiquement acceptable.

2. Utilisation des dérivés de céphalosporine de formule générale I pour la fabrication de médicaments destinés à combattre des infections bactériennes.
